Europäisches Patentamt

European Patent Office

Office européen des brevets

(11) Publication number: **0 117 064**
**B1**

(12) **EUROPEAN PATENT SPECIFICATION**

(45) Date of publication of patent specification: **08.08.90**

(51) Int. Cl.⁵: **A 61 L 2/04** // A61K35/16

(21) Application number: **84300316.1**

(22) Date of filing: **19.01.84**

(54) **Process for heat treatment of blood coagulation factor VIII.**

(30) Priority: **20.01.83 JP 8139/83**

(43) Date of publication of application:
**29.08.84 Bulletin 84/35**

(45) Publication of the grant of the patent:
**08.08.90 Bulletin 90/32**

(84) Designated Contracting States:
**BE DE FR GB NL SE**

(56) References cited:
**EP-A-0 018 561**
**EP-A-0 035 204**

The file contains technical information
submitted after the application was filed and
not included in this specification

(73) Proprietor: **GREEN CROSS CORPORATION**
**15-1, Imabashi-1-chome Higashi-ku Osaka-shi**
**Osaka 541 (JP)**

(72) Inventor: **Kameyama, Shoju**
**5D-15-505, Otokoyama-Sasatani**
**Yawata-shi (JP)**
Inventor: **Akira, Toshiaki**
**19-3, Yamatedai-6-chome**
**Ibaraki-shi (JP)**
Inventor: **Okano, Kanemichi**
**2-3-610, Minase-2-chome Shimamotocho**
**Mishima-gun Osaka (JP)**
Inventor: **Iga, Yoshiro**
**9-33, Danjocho-1-chome**
**Nishinomiya-shi (JP)**
Inventor: **Hasegawa, Eiichi**
**12-15, Hoshigaoka-2-chome**
**Hirakata-shi (JP)**

(74) Representative: **Harrison, David Christopher**
**et al**
**MEWBURN ELLIS & CO 2/3 Cursitor Street**
**London EC4A 1BQ (GB)**

Courier Press, Leamington Spa, England.

**Description**

This invention relates to a process of heat treatment to inactivate the viruses suspected of contaminating an aqueous solution or fraction containing human blood coagulation factor VIII.

The blood coagulation factor VIII (hereinafter referred to briefly as factor VIII), also called antihemophilia factor A, is one of the blood coagulation factors contained in human plasma. A diathesis due to congenital deficiency of the factor VIII is a disease called hemophilia A. In a patient suffering from this disease, the blood coagulation reaction necessary in the event of hemorrhage will not become complete and even a slight wound leads to a large amount of bleeding.

The factor VIII preparations are widely in use for the purpose of treatment and prevention of hemorrhage by supplying the factor VIII to the patients suffering from congenital deficiency or diminution of factor VIII. In recent years, however, the onset of serum hepatitis accompanied with the transfusion of blood or blood components has become one of the serious social problems. It has been made clear that the cause for the serum hepatitis is a hepatitis virus. The human serum protein preparations prepared by the fractionation of blood plasma also involves the problem of hepatitis incidence. The factor VIII, which is the subject of this invention, is also one of the human serum protein preparations and is suspected of contamination with hepatitis viruses.

It was found in an effort to solve the problem of hepatitis-viral infection that the infective activity of hepatitis viruses in serum preparations, in general, particularly in albumin preparations, may be controlled by heat treatment at 60°C for 10 hours without causing denaturation of the albumin. Since albumin preparations which have undergone such a heat treatment have been clinically used as a drug with safety, the heat treatment at 60°C for 10 hours is now being adapted to other human serum protein preparations. In order to apply such a heat treatment, the substance being treated must, of course, be stable to the treatment. The factor VIII, which is the subject of this invention, however, loses its activity to a marked degree when it is heated in an aqueous solution at 60°C for 10 hours.

Regarding the heat treatment of factor VIII, H. J. Weiss et al. reported in 1965 that a citrated plasma adjusted to pH 6.9 retained 90% of its activity of factor VIII after the plasma had been heated at 37°C for 18 hours (Thromb. Diath. Haem., Vol. 14, p. 32). More recently, two reports were published one after another on a process of heat-treating factor VIII in a solution in the presence of a sugar, namely JP—A—145615/80 and EP—A—0035204. In the heat treatment processes described in these documents, a necessary condition for heat treatment is the presence of saccharose alone or in combination with an amino acid in a concentration of 20 to 60% (W/W) in terms of saccharose.

The present invention is predicted upon the finding that in heat-treating factor VIII to inactivate hepatitis viruses, the heat stability of said factor is improved to a marked degree by using as a stabilizer a sugar-alcohol, namely sorbitol, in a high concentration.

An object of the present invention is to provide a novel process of heat treatment wherein the thermal stability of the human blood coagulation factor VIII during heat treatment for inactivation of any hepatitis viruses contaminating an aqueous solution or fraction containing the factor VIII is improved.

Other objections and advantages of the present invention will become apparent from the description which follows.

According to the present invention, there is provided a process for the heat treatment of factor VIII to inactivate hepatitis viruses suspected of contamination, which comprises heating a solution or fraction containing said factor VII, preferably at 30° to 80°C for 3 to 24 hours or at 90°C for 1 minute, in the presence of sorbitol as a stabilizer, the sorbitol being present in a high concentration of 1.5 g or more per ml of said aqueous solution or fraction containing the factor VIII.

The factor VIII to be treated according to this invention is subject to no restriction so long as it is of human origin. Factor VIII is contained chiefly in the human plasma and the methods for its separation and purification using human plasma as the starting material are already known (US—A—3,631,018, PEG fractionation method; US—A—3,652,530, Glycine fractionation method; Japanese Patent Publication No. 1290/1980, Anion-exchange treatment method; Johnson A. J. et al., British Journal of Haematology, *21*, 21 (1970), Co-use of aluminum hydroxide adsorption method and PEG fractionation method; Wagner R. B. et al., Thrombosis Diathesis Haemorrhagica, *11*, 64 (1964), Co-use of aluminum hydroxide adsorption method and PEG fractionation method).

The solution being heat-treated has a pH of generally 5.0 to 10.0, preferably 6.0 to 8.0 and the activity of factor VIII contained in the solution is preferably 1 to 50 units/ml.

A series of sample solutions were prepared by adding varied amounts of sorbitol in the range of 0.4 to 2.5 g to 1 ml of a solution containing factor VIII. Each of the resulting sample solutions was heat-treated at 60°C for 2 hours to examine the retention of the activity of factor VIII. The results obtained were as shown in Table 1. Entirely no loss of the factor VIII was observed after the said heat treatment when the sugar alcohol content is 1.5 g [corresponding to an ultimate concentration of 60% (W/W), assuming the specific gravity of the solution to be 1] or more. The assay of the activity of factor VIII was performed by the method of thromboplastin formation test [Pool and Robbinson, British Journal of Haematology, *5*, 17 (1959)].

## EP 0 117 064 B1

### TABLE 1

| | Amount of added sorbitol, g | | | | | |
|---|---|---|---|---|---|---|
| | 0.48 | 0.79 | 1.0 | 1.5 | 2.2 | 2.5 |
| Retention of activity, % | 10 | 26 | 58 | 100 | 100 | 100 |

In the next experiment, sorbitol or a mixture of sorbitol and glycine was added to 1 ml of a solution containing factor VIII. The resulting solution was subjected to heat treatment at 60°C for 10 hours to examine the retention of the activity of factor VIII. The results were as shown in Table 2.

### TABLE 2

| Stabilizer and amount added | | Retention of activity, % |
|---|---|---|
| Sorbitol | 1.5 g | 58.0 |
| Sorbitol<br>Glycine | 1.0 g<br>0.15 g | 47.5 |
| Sorbitol | 1.2 g | 43.5 |
| None | | 0 |

The correlation between the degree of purification and the thermal stability of factor VIII is insignificant. The stabilizing effect of the sorbitol remains unchanged when factor VIII of whatever degree of purification is used. As a consequence, the heat treatment for the inactivation of hepatitis viruses may be carried out at any stage of purification of factor VIII.

The process of this invention is useful as a commercial process for the production of a factor VIII preparation, because according to this invention it is possible to inactivate the hepatitis viruses suspected of contaminating a blood preparation without causing an excessive loss in the activity of factor VIII which is a valuable principle of blood preparations.

The invention is illustrated below with reference to Examples, but the invention is not limited thereto.

Example 1

A solution containing partially purified factor VIII is adjusted to pH 7.0 with 0.2 N hydrochloric acid. Into 1 ml of the solution, was added 2.0 g of sorbitol which was allowed to dissolve by heating at 37°C. The resulting solution was heated in a water bath at 60°C for 10 hours to inactivate hepatitis viruses. Substantially no precipitate was formed and, hence, the step of precipitate removal was unnecessary. The sorbitol was removed by ultrafiltration and the factor VIII was concentrated. The retention of the activity of factor VIII was found to be 58%.

Example 2

A cryoprecipitate extract originated from human plasma was adjusted to pH 7.0 with 0.2 N hydrochloric acid. Into 1 ml of the adjusted extract, was added 2.0 g of sorbitol and allowed to dissolve by heating in a bath at 37°C. The resulting solution was heated in a bath at 80°C for 3 hours to inactivate hepatitis viruses. The sorbitol was removed by ultrafiltration and the factor VIII was concentrated. The retention of the activity of factor VIII was 58%.

In the foregoing Example 2, there was no formation of precipitate and, accordingly, the operation of precipitate removal was not needed.

**Claims**

1. A process for heat treatment of a solution or fraction containing blood coagulation factor VIII so as to inactivate viruses suspected of contaminating the solution or fraction, which process comprises carrying out the heat treatment in the presence of a stabilizer, characterised in that the stabilizer is sorbitol in a concentration of 1.5 g or more per ml of said solution or fraction.

2. A process according to claim 1, wherein the concentration of sorbitol is 2 g or more per ml of said solution or fraction.

3. A process according to claim 1 or 2, wherein the heat treatment is carried out at 30° to 80°C for 3 to 24 hours or at 90°C for 1 minute.

3

# EP 0 117 064 B1

## Patentansprüche

1. Verfahren zur Hitzebehandlung einer den Blutgerinnungsfaktor VIII enthaltenden Lösung oder Fraktion, so daß die der Verunreinigung der Lösung oder Fraktion verdächtigen Viren inaktiviert werden, wobei das Verfahren das Durchführen der Hitzebehandlung in Gegenwart eines Stabilisators umfaßt, dadurch gekennzeichnet, daß der Stabilisator Sorbit in einer Konzentration von 1,5 g oder mehr pro ml der Lösung oder Fraktion ist.

2. Verfahren nach Anspruch 1, wobei die Sorbit-Konzentration 2 g oder mehr pro ml der Lösung oder Fraktion beträgt.

3. Verfahren nach den Ansprüchen 1 oder 2, wobei die Hitzebehandlung bei 30 bis 80°C 3 bis 24 Stunden lang oder bei 90°C 1 Minute lang durchgeführt wird.

## Revendications

1. Procédé pour le traitement par la chaleur d'une solution ou d'une fraction contenant le facteur VIII de coagulation du sang de manière à inactiver des virus soupçonnés de contaminer la solution ou la fraction, lequel procédé comprend le fait d'effectuer le traitement par la chaleur en présence d'un stabilisant, caractérisé en ce que le stabilisant est le sorbitol à une concentration de 1,5 g ou davantage par ml de cette solution ou fraction.

2. Procédé selon la revendication 1, dans lequel la concentration du sorbitol est de 2 g ou davantage par ml de cette solution ou fraction.

3. Procédé selon la revendication 1 ou 2 dans lequel le traitement par la chaleur est effectué à 30 à 80°C pendant 3 à 24 heures ou à 90°C pendant 1 minute.